(19)

**Europäisches Patentamt**
**European Patent Office**
**Office européen des brevets**

(11)  **EP 3 761 317 A1**

(12)  **EUROPEAN PATENT APPLICATION**

(43) Date of publication:
06.01.2021  Bulletin 2021/01

(51) Int Cl.:
*G16H 30/40* (2018.01)

(21) Application number: 19183802.8

(22) Date of filing: 02.07.2019

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventor: **WANG, Wenjing**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54)  **DEVICE, METHOD AND SYSTEM FOR REGISTERING A FIRST IMAGE FRAME AND A SECOND IMAGE FRAME**

(57)   The present invention relates to a device (150) for registering a first image frame (120) acquired by a first imaging unit (110) and a second image frame (140) acquired by a second imaging unit (130), both the first and the second image frames (120, 140) depicting a common region of interest (160), the device (150) comprising a processing unit (190) configured to measure a first pixel displacement (200) between the first image frame (120) and the second image frame (140), to correct the first pixel displacement (200) according to spatial and/or temporal geometric constraints between the first imaging unit (110) and the second imaging unit (130), and to register the first image frame (120) with the second image frame (140) based on the corrected first pixel displacement (200).

FIG.1

EP 3 761 317 A1

**Description**

FIELD OF THE INVENTION

The present invention relates to a device, a method and a system for registering a first image frame and a second image frame.

BACKGROUND OF THE INVENTION

Vital signs of a person, for example the heart rate (HR), the respiration rate (RR) or the arterial blood oxygen saturation ($SpO_2$), serve as indicators of the current state of a person and as powerful predictors of serious medical events. For this reason, vital signs are extensively monitored in inpatient and outpatient care settings, at home or in further health, leisure and fitness settings.

One way of measuring vital signs is plethysmography. Plethysmography generally refers to the measurement of volume changes of an organ or a body part and in particular to the detection of volume changes due to a cardio-vascular pulse wave traveling through the body of a subject with every heartbeat.

Photoplethysmography (PPG) is an optical measurement technique that evaluates a time-variant change of light reflectance or transmission of an area or volume of interest. PPG is based on the principle that blood absorbs light more than surrounding tissue, so variations in blood volume with every heart beat affect transmission or reflectance correspondingly. Besides information about the heart rate, a PPG waveform (also called PPG signal) can comprise information attributable to further physiological phenomena such as the respiration. By evaluating the transmittance and/or reflectivity at different wavelengths (typically red and infrared), the blood oxygen saturation can be determined.

Conventional pulse oximeters (also called contact PPG device herein) for measuring the heart rate and the (arterial) blood oxygen saturation of a subject are attached to the skin of the subject, for instance to a fingertip, earlobe or forehead. Therefore, they are referred to as 'contact' PPG devices. Although contact PPG is regarded as a basically non-invasive technique, contact PPG measurement is often experienced as being unpleasant and obtrusive, since the pulse oximeter is directly attached to the subject and any cables limit the freedom to move and might hinder a workflow.

Non-contact, remote PPG (rPPG) devices (also called camera-based device) for unobtrusive measurements have been proposed in the last decade. Remote PPG utilizes light sources or, in general radiation sources, disposed remotely from the subject of interest. Similarly, also a detector, e.g., a camera or a photo detector, can be disposed remotely from the subject of interest. Therefore, remote photoplethysmographic systems and devices are considered unobtrusive and well suited for medical as well as non-medical everyday applications. Remote PPG is e.g. disclosed in W. Wang, A. C. den Brinker, S. Stuijk, and G. de Haan, "Algorithmic principles of remote-PPG," IEEE Transactions on Biomedical Engineering, vol. 64, no. 7, pp. 1479 - 1491, 2017 and M. Van Gastel, S. Stuijk, and G. De Haan, "New principle for measuring arterial blood oxygenation, enabling motion-robust remote monitoring," Scientific reports, vol. 6, p. 38609, 2016.

Using PPG technology, vital signs can be measured, which are revealed by minute light absorption changes in the skin caused by the pulsating blood volume, i.e. by periodic color changes of the human skin induced by the blood volume pulse. As this signal is very small and hidden in much larger variations due to illumination changes and motion, there is a general interest in improving the fundamentally low signal-to-noise ratio (SNR). Thus, such an improved PPG signal should be free from distortions such as body motions, light spectra variations, low skin pulsatility and/or non-skin pixels pollution, etc. There still are demanding situations, with severe motion, challenging environmental illumination conditions, or high required accuracy of the application, where an improved robustness and accuracy of the vital sign measurement devices and methods is required, particularly for the more critical healthcare applications.

Video Health Monitoring (Heart Rate, respiration rate, SpO2, actigraphy, delirium, etc.) is a promising emerging field. Its inherent unobtrusiveness has distinct advantages for patients with fragile skin, or in need of long-term vital signs monitoring, such as NICU patients, patients with extensive burns, or COPD patients who have to be monitored at home during sleep. In other settings such as in a general ward or emergency department, the comfort of contactless monitoring is still an attractive feature.

While a promising new field, many challenges have to be overcome. Designing the system to be robust to movements of the patient is currently one of the main challenges, particularly to enable application in the emergency department.

A means for providing such a robust system for vital signs extraction (e.g., pulse extraction) is, for example, a combination of multi-wavelength channels to eliminate (motion-) distortions from a measured signal. Therefore, a system may have a multi-spectral camera equipped with Bayer filters to measure the skin at different wavelengths. However, Bayer filters are commonly available as RGB filters but less available as near infrared (NIR-) filters, which NIR wavelengths are particularly advantageous for pulse extraction. Furthermore, multi-spectral camera systems are still considered to be expensive which may be unchanged in a mid-term perspective (i.e. 5 years) and therefore restricts their broad application.

Elsewise, in order to improve overall system flexibility, multiple monochrome cameras can be used having optical filters for predefined wavelengths or wavelength ranges. Such systems provide a cost effective alternative compared to multi-spectral camera systems and additionally allow a higher degree of freedom for wavelengths selection (e.g., 760 nm, 800 nm, 905 nm) compared to multi-spectral camera systems, since they are independent from the availability of, i.e., Bayer filters. Additionally, multiple monochrome camera systems can be applied with narrow band filters which enable $SpO_2$ measurement. In contrast, multi-spectral camera systems usually have cross-talk over bands that do not allow $SpO_2$ measurement.

Multiple monochrome camera systems commonly comprise two or more cameras spaced apart from one another viewing at a common region of interest. Since the two or more cameras are spaced apart, their optical paths are different when focusing the common region of interest. This leads to a displacement in position of the common region of interest when viewing at two image frames acquired from the two or more cameras, wherein the occurring phenomenon is commonly referred to as "parallax". Since the significance of parallax on the measurement for vital signs extraction depends, among others, on focal length and a distance between the region of interest and the camera(s), it can still be considered as a significant challenge for the use of multiple monochrome cameras.

An existing solution for the reduction of parallax is image registration / alignment. Thereby, image registration commonly comprises two stages, wherein in a first stage, a calibration is performed (e.g., for the first few frames of recording) in order to estimate a linear transformation model (such as translation, Euclidean, affine or homography) between image frames of different cameras. In a second stage, the estimated model may be applied to subsequent image frames to register said image frames to a reference image frame.

US 2018/0262685 A1 discloses a system that addresses the problem of parallax occurring when shooting, e.g., a 360° video using multiple lenses cameras. The system uses stitching of image frames from multiple cameras. A warp transformation is applied to determine a displacement of pixels of a border region of an acquired area. Furthermore, spatial and/or temporal smoothing is applied and the warp transformation is determined at multiple spatial scales.

SUMMARY OF THE INVENTION

It is an object of the present invention to provide a device, a method and a system that reduce the impact of parallax on the registration of image frames.

In a first aspect of the present invention a device for registering a first image frame acquired by a first imaging unit and a second image frame acquired by a second imaging unit is presented, both the first and the second image frames depicting a common region of interest, the device comprising a processing unit configured to measure a first pixel displacement between the first image frame and the second image frame, to correct the first pixel displacement according to spatial and/or temporal geometric constraints between the first imaging unit and the second imaging unit, and to register the first image frame with the second image frame based on the corrected first pixel displacement.

In a further aspect of the present invention, a system is presented which comprises a first imaging unit configured to acquire a first image frame, a second imaging unit spaced apart from the first imaging unit and configured to acquire a second image frame, and a device for registering the first image frame and the second image frame according to the present invention.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the idea of using a non-linear adaptive image registration to solve the above-mentioned problems of conventional image registration.

According to the present invention, the processing unit involves measuring a displacement of pixels or a group of pixels between, for example, two different image frames preferably acquired by two different imaging units (i.e. cameras) at the same time, wherein the two image frames depict the same object or region of interest, respectively. The measured pixel displacement is preferably used to interpolate image frames in order to have the same alignment. Furthermore, spatial and/or temporal constraints of different image frames are used to restrict the measurement of pixel-to-pixel displacement or a displacement between a predefined group of pixels in order to smooth the interpolation. Thus, correction of the measured pixel displacement can either be based on solely spatial constraints, solely temporal constraints or both spatial and temporal (spatio-temporal) constraints jointly. Preferably, in a case where temporal constraints are exploited, a time window size may be defined. Thereby, the term "time window size" may be understood as the time window length for a buffer of images. Thus, when a temporal image sequence is used to estimate the temporal constraints of a model, temporal image sequence may be set to at least two images in time (t → t+1), but may be alternatively set to a couple of images in time (t → t+N).

The present invention has the advantage and strength that it allows non-linear registration between different imaging units. Such non-linear registration is adaptive to video content and can therefore be used to improve registration of image frames acquiring an object in motion. Thereby, the image registration can be applied for scenes with depth changes or subject / object movements in an acquired scene (e.g., changes of a distance of the object to be measured to a respective imaging unit).

Thus, the present invention shows a clear advantage in comparison to transformation based image registration (as e.g. disclosed in US 2018/0262685 A1), since transformation based image registration is linear and therefore has clear limitations when a scene with clear depth information and/or objects with 3D geometries is to be measured, as it is only effective for 2D planes.

Furthermore, transformation based image registration uses estimation of a registration model which is not adaptive to video contents, since such a registration model is only valid for a subject (e.g., face) with the fixed distance-to-camera used for the model estimation. If the subject moves and changes its distance-to-camera during the measurement, the transformation based registration results are erroneous and may introduce, for example, color gradients and/or artifacts to vital signs extraction signal considered to be harmful for health signal extraction, especially for extraction methods such as blood volume pulse vector (PBV) method (as e.g. disclosed in G. de Haan, A. van Leest, "Improved motion robustness of remote-PPG by using the blood volume pulse signature", Physiol. Meas., vol. 35, no. 9, pp. 1913-1922, Oct. 2014), Chrominance-based method (as e.g. disclosed in G. de Haan and V. Jeanne, "Robust Pulse Rate from Chrominance-Based rPPG," in IEEE Transactions on Biomedical Engineering, vol. 60, no. 10, pp. 2878 - 2886, Oct. 2013) and plane-orthogonal-to-skin (POS) method (as e.g. disclosed in W. Wang, A. C. den Brinker, S. Stuijk, and G. de Haan, "Algorithmic principles of remote-PPG," IEEE Transactions on Biomedical Engineering, vol. 64, no. 7, pp. 1479 - 1491, 2017). In particular, measurement of blood oxygen saturation depends on the amplitude of color changes and is therefore highly sensitive to registration artifact. With the present invention, this can be avoided, since the registration model is adaptive, i.e. to video content.

Especially in a setup with large parallax between imaging units, clear scene depth, and/or object motions (where distance between object and imaging unit varies in time), the non-linear and adaptive image registration is considered to be advantageous.

Furthermore, image registration according to the present invention can effectively reduce color artifacts normally caused by imperfect image registration that are well known for linear-based image registration.

In addition, in health monitoring systems where an integrated solution (e.g., having a single optical path) is not common and/or too expensive, the present invention is considered to be beneficial, since a health monitoring system comprising the device according to the present invention and multiple imaging units, preferably multi-spectral NIR cameras, can be used as an effective alternative, since quality of image registration between the multiple imaging units can be significantly improved. Thus, robustness/accuracy of vital signs extraction, especially of $SpO_2$ monitoring that preferably uses multiple NIR wavelengths for calibration, can be improved.

In a further embodiment of the device according to the present invention, the first image frame and the second image frame are acquired at a same point in time. Thereby, it is not only preferable that the first and the second image frames used for registration are acquired by the two imaging units at the same time, but also that the two imaging units are synchronized to one another.

In a yet further embodiment of the device according to the present invention, the processing unit is configured to measure, as the first pixel displacement, a pixel-to-pixel displacement between pixels or a displacement between a group of pixels inside the region of interest. The measurement of the pixel displacement is preferably done by firstly selecting the first or the second image frame as a reference image frame. Secondly, a displacement of each pixel or a predefined group of pixels between the reference image frame and the non-reference image frame is measured. Especially in cases where accuracy requirements are reduced, e.g. due to redundant information of measurement data, measurement of displacement of a group of pixels inside the region of interest instead of pixelwise displacement can show advantages, since computing time can be reduced and performance of the registration can be increased.

In a further embodiment of the device according to the present invention, the processing unit is configured to measure the first pixel displacement based on a dense optical flow acquired for each individual pixel inside the region of interest or for a group of pixels inside the region of interest. The term "dense optic flow" refers to a pattern of an apparent motion of an object, a surfaces and/or edges between two image frames, either acquired at the same time by two different imaging units spaced apart from each other (parallax), or acquired at two different times by one imaging unit (i.e. when the object is in motion). Furthermore, dense optical flow can be defined as a distribution of apparent velocities of movement of pixels or a group of pixels between two different image frames.

In a yet further embodiment of the device according to the present invention, the dense optical flow is based on one of the Lukas Kanade flow, the Farneback flow, the Horn-Schunck flow, the block-matching flow, the deep-nets flow and/or the 3DRS flow. Thereby, the main differences between these optical flow measurement methods are their accuracy and robustness in finding pixel-matches and pixel displacements, respectively, wherein higher accuracy leads to higher costs for the implementation of the respective dense optical flow measurement method. Furthermore, the different dense optical flow measurement methods differ in terms of efficiency (e.g. 3DRS is fast in computation). For example, some of the above-mentioned optical flow measurement methods are more applicable to plain regions in image frames having fewer textures; others (namely more advanced/recent dense optical flow measurement method) are more applicable to featureless image regions.

In a further embodiment of the device according to the present invention, the processing unit is further configured to analytically calculate a second pixel displacement based on the spatial geometric constraints and/or the temporal geometric constraints and to smooth the first pixel displacement by calculating a mean value of the first pixel displacement and the second pixel displacement. Thus, for example, the value of the first pixel displacement can be refined based on predetermined spatial and/or temporal constraints, resulting in improved image registration. In particular, imprecision of registered pixels or a group of pixels in the region of interest can be minimized which improves evaluation accuracy of health related parameters.

In a yet further embodiment of the device according to the present invention, the processing unit is further configured to analytically calculate a second pixel displacement based on the spatial geometric constraints and/or the temporal geometric constraints, to detect outliners in the measured first pixel displacement by comparing said first pixel displacement with the second pixel displacement and to correct the first pixel displacement by rejecting the detected outliners. According to this embodiment of the present invention, outliners resulting from imprecise registration, i.e. caused by parallax of subject motion, can be rejected and therefore do not cause measurement inaccuracy in the measurement of health parameters. In other words, the outliners that have been removed are not used in the analysis of the health parameters. Instead of measurement data related to the outliners, an average value (i.e. mean value) of pixel or group of pixels related measurement data of pixels of group of pixels adjacent to the rejected outliners may preferably be used for measurement data analysis.

In a further embodiment of the device according to the present invention, the processing unit is configured to downscale the first image frame and the second image frame. Thereby, the term "downscaling" refers to downsize the resolution of an image frame, for example resizing the image frame from 1240x720 pixels to 640x480 pixels. Preferably, downscaling is done by spatial averaging of pixels. Advantages of downscaling are noise reduction (i.e. reduction of a camera sensor noise) and a generation of a more stable pixel representation (e.g. super pixel). Downsizing can be considered as similar to a block-to-block (group of pixels) local registration, rather than to a pixel-to-pixel local registration.

In a further embodiment of the device according to the present invention, the processing unit is configured to upscale the first pixel displacement. Thus, the considered image frame is firstly downscaled (e.g. from 1240x720 pixels to 640x480 pixels). Afterwards displacement vectors of downsized pixels or a group of downsized pixels are estimated for the downsize image frame (640x480 pixels) and the estimated displacement vectors are upscaled by multiplying them with a ratio (from 640x480 pixels to 1240x720 pixels), respectively. Then, the image frame using upscaled vectors in the original resolution is registered. Especially, the step of downscaling improves the speed of dense optical flow measurement and thus enhances overall registration efficiency.

In a yet further embodiment of the device according to the present invention, the spatial geometric constraints are based on predetermined geometric constraints between the first imaging unit and the second imaging unit. The spatial constraints may be measured using intrinsic parameters of the imaging units (i.e. imaging unit position, view angle, distance, etc.) and/or based on a measurement of image content. Thereby, corner points or features represented in the image content may be used for referencing in order to derive spatial constraints. As an example, a view relation of a 3D object acquired by a multi-camera system having at least two cameras which are spaced apart from one another (and thus having a stereo vision on the 3D object) may be used as a spatial constraint. Instead, for example, a view relation of 3D motion in such a multi-camera system may be used as a temporal constraint.

In a further embodiment of the system according to the present invention, the first imaging unit included in the system is a monochrome camera and/or a multi-spectrum camera and the second imaging unit included in the system is a monochrome camera and/or a multi-spectrum camera. Especially in a case where $SpO_2$ measurement is performed based on three different wavelengths, a system may comprise one monochrome camera and one multi-spectrum camera, wherein said cameras are spaced apart from one another. In such a case, the monochrome camera may be configured to acquire a first wavelength or wavelength range, whereas the multi-spectrum camera may be configured to acquire both a second wavelength or wavelength range and a third wavelength or wavelength range. Thus, three different wavelengths can be measured by two different cameras which lead to a reduction of cost and overall system's complexity.

In a further embodiment of the system according to the present invention, the first imaging unit included in the system is configured to acquire a first wavelength or wavelength range in the visible or infrared wavelength range and the second imaging unit included in the system is configured to acquire a second wavelength or wavelength range, different from the first wavelength or wavelength range, in the visible or infrared wavelength range. For example, both the first and the second wavelength or wavelength range may be a NIR wavelength or wavelength range.

In yet a further embodiment of the system according to the present invention, the system further comprising a health parameter extraction unit configured to extract vital signs of a subject based on the registered image frame. Thereby, the registered image frame obtained by the health parameter extraction unit results from the registration method according to the present invention which is performed by the device. With the use of the registered image frames for vital signs extraction, the impact of parallax on vital signs extraction can be reduced. According to the embodiment of the present invention, especially vital signs extraction from image frames of 3D subjects having depth information shows higher accuracy compared to state of the art vital signs extraction, i.e. based on transformation based image registration.

BRIEF DESCRIPTION OF THE DRAWINGS

These and other embodiment of the invention will be apparent from and elucidated with reference to the embodiment(s) described hereinafter. In the following drawings

Fig. 1 shows a schematic diagram of a system according to the present invention;
Fig. 2 shows a flowchart of a method according to the present invention;
Figs. 3A, 3B, 3C, 3D show a system setup (Fig. 3A) and resulting image frames (Figs. 3B, 3C, 3D) acquired by a system according to the present invention;
Fig. 4 shows a schematic diagram of a first group of image frames acquired by a system according to the present invention;
Figs. 5A, 5B show a schematic diagram of a second group of image frames acquired by a system according to the present invention;
Fig. 6 shows a schematic diagram of a third group of image frames acquired by a system according to the present invention;
Figs. 7A, 7B, 7C show image frames with no image registration (Fig. 7A), transformation based image registration (Fig. 7B) and image registration according to the present invention (Fig. 7C);
Figs. 8A, 8B, 8C show image frames with no image registration (Fig. 7A), transformation based image registration (Fig. 7B) and image registration according to the present invention (Fig. 7C); and
Figs. 9A, 9B show a comparison of a state of the art registration (Fig. 9A) and image registration according to the present invention (Fig. 9B).

DETAILED DESCRIPTION OF EMBODIMENTS

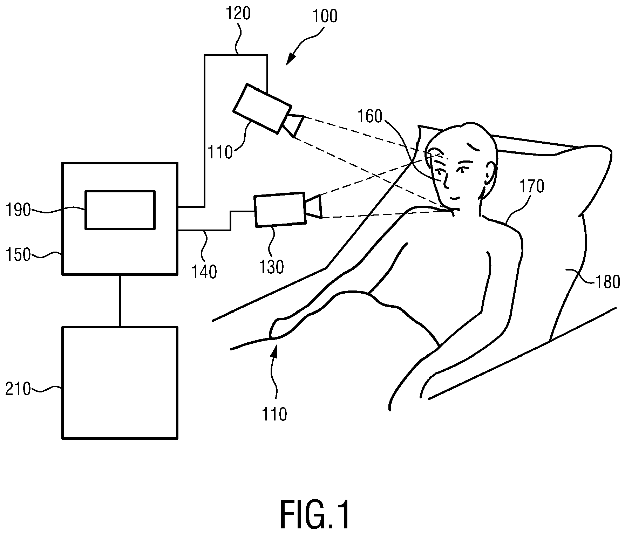

Fig. 1 shows a schematic diagram of an embodiment of a system 100 according to the present invention. The system 100 comprises a first imaging unit 110 configured to acquire a first frame 120 and a second imaging unit 130 configured to acquire a second image frame 140.
The system 100 further comprises a device 150 for registering the first image frame 120 acquired by the first imaging unit 110 and the second image frame 140 acquired by the second imaging unit 130. The imaging units 110, 130 may also be referred to as camera-based or remote PPG sensors. Both the first image frame 120 and the second image frame 140 depicting a common region of interest 160 of a subject 170. Both image frames 120, 140 include information used to determine physiological information indicative of at least one vital sign of the subject 170.
The subject 170 may be a patient, in this example a patient lying in a bed 180, e.g. in a hospital or other healthcare facility, but may also be a neonate or premature infant with very sensitive skin in NICU's, e.g. lying in an incubator, a patient with damaged (e.g. burned) skin or a person at home or in a different environment.
There exist different embodiments for a device for registering image frames acquired by different imaging units depicting a common region of interest of a subject's body, which may alternatively (which is preferred) or together be used. In the embodiment of the system 100, one exemplary embodiment of the device 150 is shown and will be explained below.
In one embodiment of the system 100, the first imaging unit 110 is a first camera and the second imaging unit is a second camera. Here, the first camera 110 is a monochrome camera and the second camera 130 is a multi-spectrum camera. In other embodiments, both the first and the second imaging unit 110, 130 may be a monochrome camera and/or a multi-spectrum camera. Preferably, the first imaging unit 110 (the first camera 112) is configured to acquire a first wavelength (such as red light at 700 nm) or wavelength range (such as red light from 680 nm to 720 nm) or infrared wavelength range (above 790 nm), whereas the second imaging unit 130 (the second camera 130) is configured to acquire a second wavelength (such as green light at 550 nm) or wavelength range (such as green light from 530 nm to 570 nm). The second wavelength or wavelength range is preferably different from the first wavelength or wavelength range.
In other embodiments, the system may comprise more than two imaging units spaced apart from one another. For example, according to a preferred embodiment, the system may further comprise a third imaging unit depicting the common region of interest 160 of a subject 170. The third imaging unit is preferably configured to acquire a third image frame. Preferably, the third imaging unit is configured to acquire a third wavelength or wavelength range which is preferably different from the first and the second wavelength or wavelength range.

Both the first camera 110 and the second camera 130 preferably include a suitable photosensor for (remotely and unobtrusively) capturing image frames (such as the first image frame 120 and the second image frame 140) of the region of interest 160 of the subject 170, in particular for acquiring a sequence of image frames of the subject 170 over time, from which photoplethysmography signals can be derived. The image frames captured by the cameras 110, 130 may particularly correspond to a video sequence captured by means of an analog or digital photosensor, e.g. in a (digital) camera. Such cameras 110, 130 usually includes a photosensor, such as a CMOS or CCD sensor, which may also operate in a specific spectral range (visible, IR) or provide information for different spectral ranges. The cameras 110, 130 may provide an analog or digital signal.

The image frames 120, 140 include a plurality of image pixels having associated pixel values. Particularly, the image frames 120, 140 include pixels representing light intensity values captured with different photosensitive elements of a photosensor. These photosensitive elements may be sensitive in a specific spectral range (i.e. representing a specific color or pseudo-color (in NIR)). The image frames 120, 140 include at least some image pixels being representative of a skin portion of the subject 170. Thereby, an image pixel may correspond to one photosensitive element of a photo-detector and its (analog or digital) output or may be determined based on a combination (e.g. through binning) of a plurality of the photosensitive elements.

In some embodiments, the system 100 may further comprise a light source (also called illumination source), such as a lamp, for illuminating the region of interest 160, such as the skin of the subject's 170 face (e.g. part of the cheek or forehead), with light, for instance in predetermined wavelengths or wavelength ranges (e.g. in the red, green and/or infrared wavelength range(s)). The light reflected from said region of interest 160 in response to said illumination may be detected by the cameras 110, 130. In another embodiment no dedicated light source is provided, but ambient light is used for illumination of the subject 170. From the reflected light, only light in a number of desired wavelength ranges (e.g. green and red or infrared light, or light in a sufficiently large wavelength range covering at least two wavelength channels) may be detected and/or evaluated by the cameras 110, 130. Therefore, the cameras 110, 130 may be applied with optical filters which are preferably different, though their filter bandwidth can be overlapping. It is sufficient if their wavelength-dependent transmission is different.

The device 150 according to one aspect of the invention comprises a processing unit 190 which may be a processor of a computational device, system-on-a-chip or any other suitable unit for data processing. The processing unit 190 according to the embodiment shown in Fig. 1 is located inside the device 150, but may also be located outside, i.e., spaced apart from the device 150 and connected though one or more cables or wirelessly to device 150.

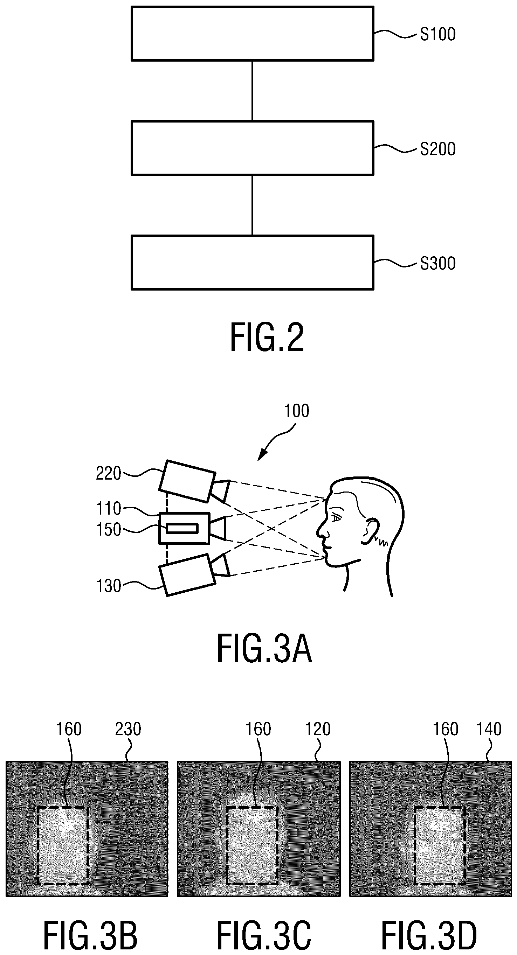

The processing unit 190 is configured to measure a first pixel displacement 200 between the first image frame 120 and the second image frame 140 and thereby executes step S100 of the method according to another aspect of the present invention (see Fig. 2). Furthermore, the processing unit 190 is configured to correct the first pixel displacement 200 according to spatial and/or temporal geometric constraints between the first imaging unit 110 and the second imaging unit 130, thereby executing step S200 of the method according to the present invention. Then, the processing unit 190 is configured to register the first image frame 120 with the second image frame 140 based on the corrected first pixel displacement 200 by executing step S300 of the invention method (see Fig. 2). It should be noted that the method may comprise one or more intermediate steps which may precede or follow the above steps S100, S200, S300, respectively.

In order to monitor health related parameters based on the registered image frames, information included in the image frames, namely pixel based or group of pixels based color or grayscale information, may be extracted from image frames in time sequence. Therefore, a health parameter extraction unit 210 may be connected to the device 150 via one or more cables or wirelessly or may be integrated in the device 150. The health parameter extraction unit 210 may be preferably configured to extract one or more health related parameters from registered successive images frames (such as the image frames 120, 140).

A system 100 as illustrated in Fig. 1 may, e.g., be located in a hospital, healthcare facility, elderly care facility or the like. Apart from the monitoring of patients, the present invention may also be applied in other fields such as neonate monitoring, general surveillance applications, security monitoring or so-called lifestyle environments, such as fitness equipment, a wearable, a handheld device like a smartphone, or the like. The uni- or bidirectional communication between the device 150 and the imaging units 110, 130 may work via a wireless or wired communication interface. Other embodiments of the present invention may include a device 150, which is not provided stand-alone, but integrated into at least one of the imaging units 110, 130.

In general, contactless monitoring may be more convenient than monitoring with contact sensors which is still used in a general ward or a triage in an emergency department. In addition, such contactless monitoring may be applicable for monitoring of automotive drivers as well as for sleep monitoring, wherein in the latter, especially NIR-based monitoring, preferably multi-spectral NIR-based monitoring, may be applied to improve robustness of vital signs extraction.

Fig. 3A shows a schematic diagram of a second embodiment the system 100 further comprising a third imaging unit 220. In this embodiment, the device 150 is integrated in the first imaging unit 110. The third imaging unit is configured to acquire a third image frame 230 depicting the common region of interest 160. The second and the third imaging unit 130, 220 are communicatively connected to the first imaging unit 110. In this case, also the third imaging unit 220 is a camera 220. Thus, the system 100 setup represents a multi-spectrum camera setup consisting of three cameras 110, 130, 220 that are spaced apart from one another depicting the same region of interest 160 viewing at the same direction (see in Figs. 3B, 3C, 3D). Preferably, the cameras 110, 130, 220 are mono-chrome cameras with optical filters to sample desired wavelengths (which may be three different NIR wavelengths). Since the cameras 110, 130, 220 have different optical paths, the region of interest of the subject / object appearing in the image frames 120, 140, 230 acquired by the cameras 110, 130, 220 have displacement in their position with respect to one another. This displacement is commonly referred to as "parallax". The significance of the parallax depends on the used focal length and the distance between the subject 170 and camera 110, 130, 220, respectively. The proposed nonlinear adaptive camera registration with spatio-temporal geometric constraints which is performed by the device 150 aims to improve the registration of the image frames 120, 140, 230 to eliminate said parallax problem.

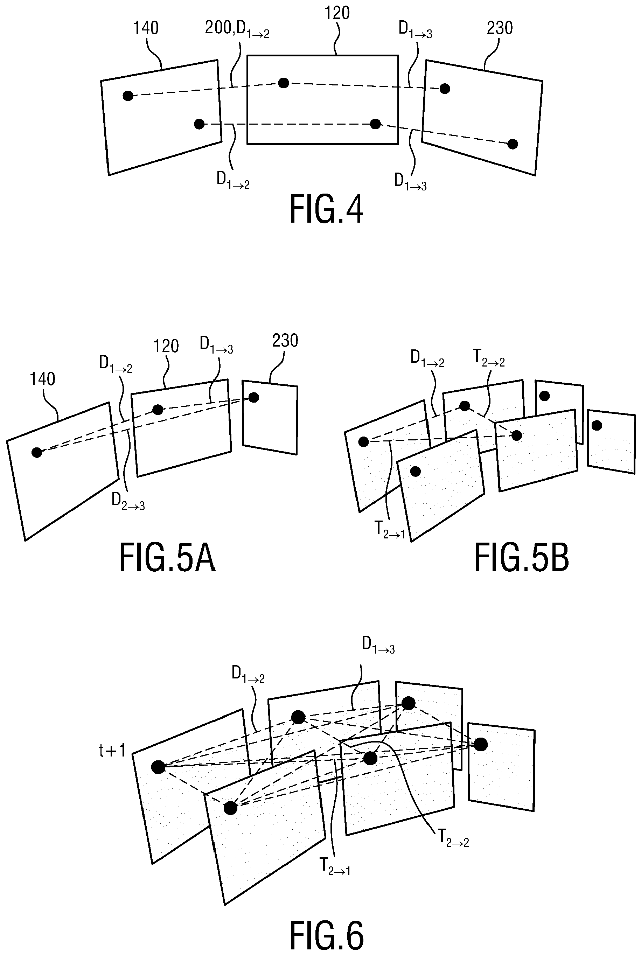

In the follow, the nonlinear adaptive image registration is explained in detail referring to Figs. 4, 5A, 5B and 6. Thereby, the image frames 120, 140, 230 have been acquired, for example, by a system according to Fig. 3A. To enable the nonlinear registration, preferably being a pixelwise registration or a registration of a predefined group of pixels across multiple cameras (here three cameras 110, 130, 220), is performed, wherein each pixel or group of pixels has its own registration in a new image frame.

Typically the image frames acquired by the central camera is taken as reference image frames. In Fig. 4, the image frame 120 acquired by the first imaging unit 110 is the reference image frame. For the image frames 140, 230 acquired by the second and the third cameras 130, 220, new image frames are created. Typically, the camera 110 placed in the central position is selected as a reference camera, since it has the shortest distance to the subject 170 compared to the cameras 130, 220. Then, the first pixel displacement 200 is measured which may be a pixel-to-pixel displacement between pixels or a displacement between a group of pixels. The first pixel displacement is measured, for example, between the first image frame 120 and the second image frame 140. Furthermore, a pixel displacement may be measured between the first image frame 120 and the third image frame 230. In Fig. 4, the first pixel displacement is measured between two pixels of the first image frame 120 relative to the second and third image frame 140, 230, respectively. The first pixel displacement may be measured by dense optical flow which results in motion vectors for pixels or group of pixels between the spatial and/or temporal image frames:

$$D = DOF(I_{ref}, I_{nonref}),$$

where DOF(.) denotes the dense optical flow, $I_{ref}$ the reference image frame (i.e. image frame 120), $I_{nonref}$ the non-reference image frames (i.e. image frames 140, 230) and D the first pixel displacement 200, wherein D is used to correlate/interpolate the non-reference image frames 140, 230 in order to register them with the reference image frame 120:

$$I_{reg} = Interp(I_{nonref}, D),$$

where Interp(.) denotes the interpolation/correlation and $I_{reg}$ the registered image. The pixel-based interpolation is highly nonlinear for image transformation and dense optical flow measurement and interpolation are performed for each individual image frame. Thus, the registration is adaptive to video contents and robust to scenes having depth changes or object position changes (e.g., distance-to-camera changes) during monitoring.

Furthermore, according to the present invention, the first pixel displacement is corrected according to spatial and/or temporal geometric constraints. These constraints are applied as a post processing step of the "raw" dense optical flow results described above. Since the setup (e.g., camera position) is preferably fixed during the measurement, parallax-induced pixel displacement across the cameras 110, 130, 220 depends on predefined geometric relationships (e.g., epipolar geometry). Thereby, epipolar geometry considers, for example, the geometry of stereo vision, wherein two cameras view a 3D scene from two distinct positions. In such a setup, there are a number of geometric relations between 3D points and their projections onto the 2D image frame leading to constraints between these image points. These relations may be derived based on the assumption that cameras can be approximated by a pinhole camera model. Such relationships may be used as spatial geometric constraints to smooth the measurement of the first pixel displacement 200 or to restrict outliners. In Fig. 5A, for example, a spatial geometric constraint between the second imaging unit 140 and the third imaging unit 220 is apparent.

Based on the spatial geometric constraint(s), a second pixel displacement can be analytically calculated. Based on the second pixel displacement, the first pixel displacement 200 may preferably be smoothed by calculating a mean value of the first pixel displacement 200 and the second pixel displacement. In other embodiments, the second pixel displacement may be used to detect outliners in the measured first pixel displacement 200 by comparing said first pixel displacement 200 with the second pixel displacement and to correct the first pixel displacement 200 by rejecting the detected outliners.

In the example shown in Figs. 4, 5A, 5B and 6, a dense optical flow D ($I_{camera\ 130}$, $I_{camera\ 220}$) = $D_{1 \to 3}$ can be measured referring to a predefined relationship between the motion vectors determined from the dense optical flow $D_{1 \to 2}$. This may be expressed as:

$$D_{1 \to 2} \text{ is the measured solution}$$

$$D'_{1 \to 2} = D_{1 \to 3} - D_{2 \to 3} \text{ (analytic solution)}$$

$$D''_{1 \to 2} = (D_{1 \to 2} + D'_{1 \to 2})/2 \text{ (smoothed solution)}$$

where $D_{1 \to 2}$, $D_{2 \to 3}$, $D_{1 \to 3}$, denote the (group of) pixel displacement from camera 110 to 130, camera 130 to 220, and camera 110 to 220, respectively. Thereby, $D_{1 \to 2}$ is the solution measured by dense optical flow, $D'_{1 \to 2}$ is the analytic solution deducted from $D_{1 \to 3}$ and $D_{2 \to 3}$, and $D''_{1 \to 2}$ is the smoothed solution resulting from the mean value between $D_{1 \to 2}$ and $D'_{1 \to 2}$. It should be noted that it may be derived, i.e. from further metrics (not described here), that instead of the "smoothed solution" the "measured" or "analytic" solution may be more appropriate. Furthermore, it is possible to use $D'_{1 \to 2}$ to restrict measurement outliers in $D_{1 \to 2}$.

Similar to the use of spatial geometric constraints, temporal geometric constraints may be used to smooth the measurement of the first pixel displacement 200 (see Fig. 5B) which may be expressed by:

$$D_{1 \to 2} \text{ is the measured solution}$$

$$D'_{1 \to 2} = T_{2 \to 1} - T_{2 \to 2} \text{ (analytic solution)}$$

$$D''_{1 \to 2} = (D_{1 \to 2} + D'_{1 \to 2})/2 \text{ (smoothed solution)}$$

where $T_{2 \to 1}$ denotes the pixel displacement from the second camera 130 at time t to the first camera 110 at time t+1 and $T_{2 \to 2}$ the pixel displacement from the first camera 110 at time t to the first camera 110 at time t+1.

Preferably, spatial and temporal geometric constraints are applied simultaneously (see Fig. 6). In such a case, all pixels of a common region of interest of an object/subject are connected in space and time for globally optimizing / correcting the pixel displacement leading to a highly robust and smooth image registration. Another benefit of using spatio-temporal geometric constraints is that the pixel motions (due to subject body motion or camera movement) between adjacent frames can be reduced.

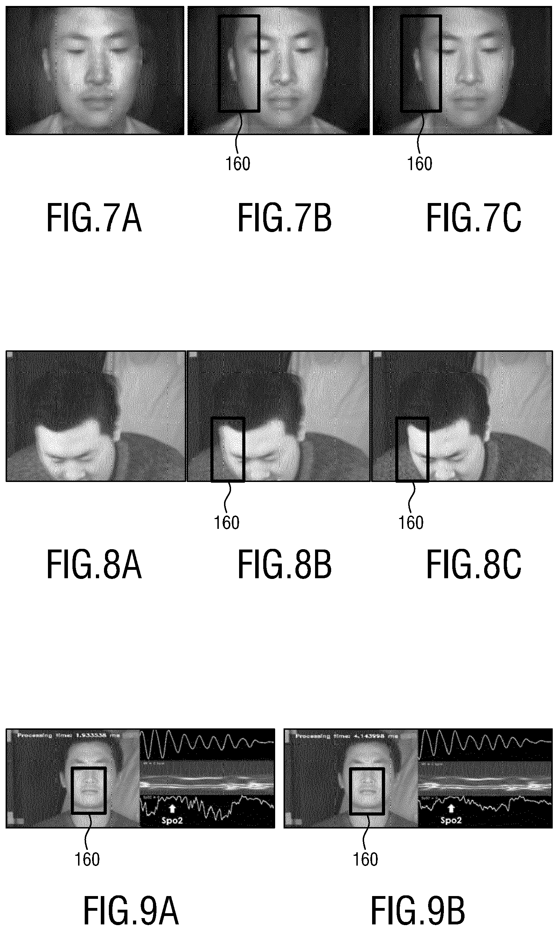

Figs. 7A, 7B, 7C show three image frames depicting a common region of interest 160 which is in this case a part of a face of a patient how's vital signs are to be measured, wherein a distance between the at least two imaging units 110, 130 and the region of interest 160 of the subject 170 is short (< 1 m) resulting in a large parallax. Instead, Figs. 8A, 8B, 8C show three image frames depicting a common region of interest 160 which is in this case a part of a face of another patient, wherein a distance between the at least two imaging units 110, 130 and the region of interest 160 of the subject 170 is large (5-6 m) resulting in a small parallax (i.e., where parallax is reduced manually).

Fig. 7A and Fig. 8A both show an unregistered image frame in which strong blurriness is recognizable due to parallax. This leads, in particular when large parallax is apparent (see Fig. 7A), to the fact that, for example, facial contours of the subject 170 are not delimitable as sharp edges / contours. Thus, the region of interest 160 may not be clearly delimited which may lead to an erroneous evaluation of health related parameters.

Fig. 7B and Fig. 8B show a case where state of the art transformation based image registration has been performed resulting in a reduction of parallax based blurriness. However, especially when large parallax is apparent (see Fig. 7B), the state of the art registration still shows a remaining blurriness at an edge area of the region of interest 160 which still results in an incorrect evaluation of health related parameters, especially when the these areas include depth information.

In Fig. 7C and Fig. 8C image registration has been performed according to an embodiment of the registration method according to the present invention resulting in an elimination of parallax based blurriness. Thus, image registration is performed resulting in sharp edges of a depicted region of interest. The improvement is particularly visible when the subject 170 changes the position or rotates the head during the monitoring, as the distance-to-camera or the face 3D geometry may change which renders the linear model used by the existing method invalid (i.e., transformation model is usually estimated upfront the measurement).

Fig. 9A shows vital signs extraction based on a state of the art registered image frames. Fig. 9B shows vital signs extraction based on inventively registered image frames. It should be noted that pulse-rate and $SpO_2$ have been extracted as exemplary vital signs and the vital signs extraction method is the same for both cases. As can be seen in the $SpO_2$ graph in Fig. 9B, curve progression is more stable when registration has been performed according to the present invention. The higher stability results from the fact that $SpO_2$ measurement is based on amplitude of color changes and is therefore rather sensitive to color gradient artefacts which can be reduced due to the image registration according to the present invention.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/ distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. A device (150) for registering a first image frame (120) acquired by a first imaging unit (110) and a second image frame (140) acquired by a second imaging unit (130), both the first and the second image frames (120, 140) depicting a common region of interest (160), the device (150) comprising a processing unit (190) configured to:

    - measure a first pixel displacement (200) between the first image frame (120) and the second image frame (140);
    - correct the first pixel displacement (200) according to spatial and/or temporal geometric constraints between the first imaging unit (110) and the second imaging unit (130); and
    - register the first image frame (120) with the second image frame (140) based on the corrected first pixel displacement (200).

2. Device according to claim 1, wherein the first image frame (120) and the second image frame (140) are acquired at a same point in time.

3. Device according to claim 1, wherein the processing unit (190) is configured to measure, as the first pixel displacement (200), a pixel-to-pixel displacement between pixels or a displacement between a group of pixels inside the region of interest (160).

4. Device according to claim 1, wherein the processing unit (190) is configured to measure the first pixel displacement (200) based on a dense optical flow acquired for each individual pixel inside the region of interest (160) or for a group of pixels inside the region of interest (160).

5. Device according to claim 4, wherein the dense optical flow is based on one of the Lukas Kanade flow, the Fameback flow, the Horn-Schunck flow, the block-matching flow, the deep-nets flow and/or the 3DRS flow.

6. Device according to any one of the preceding claims, wherein the processing unit (190) is further configured to analytically calculate a second pixel displacement based on the spatial geometric constraints and/or the temporal geometric constraints and to smooth the first pixel displacement (200) by calculating a mean value of the first pixel displacement (200) and the second pixel displacement.

7. Device according to any one of claims 1 to 5, wherein the processing unit (190) is further configured to analytically calculate a second pixel displacement based on the spatial geometric constraints and/or the temporal geometric constraints, to detect outliners in the measured first pixel displacement (200) by comparing said first pixel displacement (200) with the second pixel displacement and to correct the first pixel displacement (200) by rejecting the detected outliners.

8. Device according to any one of the preceding claims, wherein the processing unit (190) is configured to downscale the first image frame (120) and the second image frame (140) and/or to upscale the first pixel displacement (200).

9. Device according to any one of the preceding claims, wherein the spatial geometric constraints are based on predetermined geometric constraints between the first imaging unit (110) and the second imaging unit (130).

10. A system (100) comprising:

   - a first imaging unit (110) configured to acquire a first image frame (120),
   - a second imaging unit (130) spaced apart from the first imaging unit (110) and configured to acquire a second image frame (140), and
   - a device (150) according to any one of the preceding claims for registering the first image frame (120) and the second image frame (140).

11. System according to claim 10, wherein the first imaging unit (110) is a monochrome camera (110) and/or a multi-spectrum camera (110) and wherein the second imaging unit (130) is a monochrome camera (130) and/or a multi-spectrum camera (130).

12. System according to claim 10 or 11, wherein the first imaging unit (110) is configured to acquire a first wavelength or wavelength range in the visible or infrared wavelength range, and wherein the second imaging unit (130) is configured to acquire a second wavelength or wavelength range, different from the first wavelength or wavelength range, in the visible or infrared wavelength range.

13. System according to claims 10 to 12 further comprising a health parameter extraction unit (210) configured to extract vital signs of a subject (170) based on the registered image frame (120, 140).

14. A method for registering a first image frame (120) acquired by a first imaging unit (110) and a second image frame (140) acquired by a second imaging unit (130), the method comprising the steps of:

   - measuring (S100) a first pixel displacement (200) between the first image frame (120) and the second image frame (140);
   - correcting (S200) the first pixel displacement (200) according to spatial and/or temporal geometric constraints between the first imaging unit (110) and the second imaging unit (130); and
   - registering (S300) the first image frame (120) with the second image frame (140) based on the corrected first pixel displacement (200).

15. Computer program comprising program code means for causing a computer to carry out the steps of the method as claimed in claim 14 when said computer program is carried out on the computer.

FIG.1

FIG.2

FIG.3A

FIG.3B  FIG.3C  FIG.3D

FIG.4

FIG.5A

FIG.5B

FIG.6

160

160

FIG.7A          FIG.7B          FIG.7C

160

160

FIG.8A          FIG.8B          FIG.8C

160

160

FIG.9A                    FIG.9B

Europäisches
Patentamt

European
Patent Office

Office européen
des brevets

## EUROPEAN SEARCH REPORT

Application Number

EP 19 18 3802

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VAN GASTEL MARK ET AL: "Motion Robust Remote-PPG in Infrared", IEEE TRANSACTIONS ON BIOMEDICAL ENGINEERING, IEEE SERVICE CENTER, PISCATAWAY, NJ, USA, vol. 62, no. 5, 1 May 2015 (2015-05-01), pages 1425-1433, XP011578781, ISSN: 0018-9294, DOI: 10.1109/TBME.2015.2390261 [retrieved on 2015-04-17] | 1-3,7-15 | INV. G16H30/40 |
| Y | * abstract * * figures 8, 9 * * 1) Image Registration; page 1430 * | 4-6 | |
| Y | DONGXIANG ZHOU ET AL: "Modified GMM Background Modeling and Optical Flow for Detection of Moving Objects", SYSTEMS, MAN AND CYBERNETICS, 2005 IEEE INTERNATIONAL CONFERENCE ON, IEEE, PISCATAWAY, NJ, USA, vol. 3, 10 October 2005 (2005-10-10), pages 2224-2229, XP010873991, DOI: 10.1109/ICSMC.2005.1571479 ISBN: 978-0-7803-9298-4 * 2 Outline of the algorithm; page 2 * * 4 Optical flow computation; page 3 * | 4,5 | |

_____

-/--

TECHNICAL FIELDS SEARCHED (IPC)

G16H

The present search report has been drawn up for all claims

| Place of search | Date of completion of the search | Examiner |
|---|---|---|
| Munich | 19 December 2019 | Beligny, Samuel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 19 18 3802

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | VINCENT GAY-BELLILE ET AL: "Image Registration by Combining Thin-Plate Splines with a 3D Morphable Model", IMAGE PROCESSING, 2006 IEEE INTERNATIONAL CONFERENCE ON, IEEE, PI, 1 October 2006 (2006-10-01), pages 1069-1072, XP031048825, ISBN: 978-1-4244-0480-3 * 5. Conclusion; page 1072 * | 6 | |

----

TECHNICAL FIELDS
SEARCHED (IPC)

The present search report has been drawn up for all claims

| Place of search | Date of completion of the search | Examiner |
|---|---|---|
| Munich | 19 December 2019 | Beligny, Samuel |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

**EP 3 761 317 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180262685 A1

**Non-patent literature cited in the description**

- **W. WANG ; A. C. DEN BRINKER ; S. STUIJK ; G. DE HAAN.** Algorithmic principles of remote-PPG. *IEEE Transactions on Biomedical Engineering,* 2017, vol. 64 (7), 1479-1491
- **M. VAN GASTEL ; S. STUIJK ; G. DE HAAN.** New principle for measuring arterial blood oxygenation, enabling motion-robust remote monitoring. *Scientific reports,* 2016, vol. 6, 38609

- **G. DE HAAN ; A. VAN LEEST.** Improved motion robustness of remote-PPG by using the blood volume pulse signature. *Physiol. Meas.,* October 2014, vol. 35 (9), 1913-1922
- **G. DE HAAN ; V. JEANNE.** Robust Pulse Rate from Chrominance-Based rPPG. *IEEE Transactions on Biomedical Engineering,* October 2013, vol. 60 (10), 2878-2886